# EUROPÄISCHE PATENTSCHRIFT

(11) **EP 3 211 058 B1**
(45) Veröffentlichungstag und Bekanntmachung des Hinweises auf die Patenterteilung: **23.08.2023**
(21) Anmeldenummer: 17165036.9
(22) Anmeldetag: 26.07.2006
(51) Int. Cl.: C09K 11/06, C07C 13/62, C07C 13/72, C07C 25/22, C07D 237/26, C07D 403/10, C07D 209/94, C07D 409/10, C07D 407/10, C07D 235/02, C07D 249/16, C07D 307/77, C07D 285/14, C07D 271/12, C07D 401/10, C07D 409/04

(54) **NEUE MATERIALIEN FÜR ORGANISCHE ELEKTROLUMINESZENZVORRICHTUNGEN**
NEW MATERIALS FOR ORGANIC ELECTROLUMINESCENT DEVICES
NOUVEAUX MATÉRIAUX POUR DISPOSITIFS ÉLECTROLUMINESCENTS ORGANIQUES

(30) Priorität: 26.08.2005 DE 102005040411
(43) Veröffentlichungstag der Anmeldung: 30.08.2017
(62) Teilanmeldung aus: 06762829.7
(73) Patentinhaber: Merck Patent GmbH, 64293 Darmstadt (DE)
(72) Erfinder: Heil, Holger, 60316 FRANKFURT AM MAIN (DE); Buesing, Arne, 65929 Frankfurt am Main (DE); Stoessel, Philipp, 60389 FRANKFURT AM MAIN (DE); Vestweber, Horst, 34630 Gilserberg-Winterscheid (DE); Parham, Amir, 60486 FRANKFURT AM MAIN (DE)

(56) Entgegenhaltungen:
- EP-A- 1 491 568
- JP-A- 2000 003 790
- U. DAHLMANN ET AL: "ChemInform Abstract: The Diyne Reaction of 3,3'-Bis(phenylethynyl)-2,2'-bithiophene Derivatives via Rhodium Complexes: A Novel Approach to Condensed Benzo( 2,1-b:3,4-b')dithiophenes.", CHEMINFORM, Bd. 28, Nr. 18, 29. April 1997 (1997-04-29), Seiten no-no, XP055373230, DE ISSN: 0931-7597, DOI: 10.1002/chin.199718142
- LE BERRE, ANDRE: "Autoxidation of orthoquinoid indenofluorene hydrocarbons", ANN. CHIM., Bd. 2, 1957, Seiten 371-425, XP009074391, Paris
- HARVEY R G ET AL: "NEW SYNTHETIC APPROACH TO POLYCYCLIC AROMATIC HYDROCARBONS AND THEIR CARCINOGENIC OXIDEZED METABOLITES: DERIVATIVES OF BENZO[S]PICENE, BENZO[RST]PENTAPHENE, AND DIBENZO[B,DEF]CHRYSENE", JOURNAL OF ORGANIC CHEMISTRY, AMERICAN CHEMICAL SOCIETY. EASTON, US, Bd. 65, Nr. 13, 2000, Seiten 3952-3960, XP002271036, ISSN: 0022-3263

## Beschreibung

Organische Halbleiter werden für eine Reihe verschiedenartiger Anwendungen, die im weitesten Sinne der Elektronikindustrie zugerechnet werden können, entwickelt. Der Aufbau organischer Elektrolumineszenzvorrichtungen (OLEDs), in denen diese organischen Halbleiter als funktionelle Materialien eingesetzt werden, ist beispielsweise in US 4539507, US 5151629, EP 0676461 und WO 98/27136 beschrieben. Allerdings zeigen diese Vorrichtungen noch erhebliche Probleme, die einer dringenden Verbesserung bedürfen:
1. Die operative Lebensdauer ist insbesondere bei blauer Emission immer noch gering, so dass bis dato nur einfache Anwendungen kommerziell realisiert werden konnten.
2. Die verwendeten Verbindungen sind teilweise nur schwer in gängigen organischen Lösemitteln löslich, was ihre Reinigung bei der Synthese, aber auch die Reinigung der Anlagen bei der Herstellung der organischen elektronischen Vorrichtungen erschwert.
3. Einige der verwendeten Verbindungen, die ansonsten gute Eigenschaften in OLEDs zeigen, weisen keine ausreichend hohe Glasübergangstemperatur auf.

In fluoreszierenden OLEDs werden gemäß dem Stand der Technik verschiedene kondensierte Aromaten, insbesondere Anthracen- oder Pyrenderivate, als Host-Materialien vor allem für blau emittierende Elektrolumineszenzvorrichtungen verwendet, z. B. 9,10-Bis(2-naphthyl)-anthracen (US 5935721). In WO 03/095445 und in CN 1362464 werden 9,10-Bis(1-naphthyl)anthracen-Derivate für die Verwendung in OLEDs beschrieben. Weitere Anthracen-Derivate, die sich als Host-Materialien eignen, sind in WO 01/076323, in WO 01/021729, in WO 04/013073, in WO 04/018588, in WO 03/087023 oder in WO 04/018587 beschrieben. Host-Materialien, basierend auf Aryl-substituierten Pyrenen und Chrysenen, werden in WO 04/016575 beschrieben. Es ist für hochwertige Anwendungen notwendig, verbesserte Host-Materialien zur Verfügung zu haben.

Als Stand der Technik bei blau emittierenden Verbindungen kann die Verwendung bestimmter Arylvinylamine von Idemitsu genannt werden (z. B. WO 04/013073, WO 04/016575, WO 04/018587). Damit werden sehr gute Lebensdauern bei tiefblauer Emission zitiert. Allerdings sind diese Ergebnisse stark abhängig vom verwendeten Hostmaterial, so dass die zitierten Lebensdauern nicht als Absolutwerte verglichen werden können, sondern immer nur bei Einsatz in einem optimierten System. Weiterhin sind diese Verbindungen thermisch instabil und lassen sich nicht unzersetzt verdampfen, was daher einen hohen technischen Aufwand für die OLED-Herstellung erfordert und somit einen deutlichen technischen Nachteil darstellt. Einen weiteren Nachteil stellt die Emissionsfarbe dieser Verbindungen dar. Während Idemitsu tiefblaue Emission (CIE-y-Koordinaten im Bereich von 0.15-0.18) zitiert, konnten diese Farbkoordinaten nicht in einfachen Vorrichtungen gemäß dem Stand der Technik reproduziert werden. Im Gegenteil erhält man hier grünblaue Emission. Es ist nicht offensichtlich, wie mit diesen Verbindungen tatsächlich blaue Emission erzeugt werden kann. Es ist für hochwertige Anwendungen notwendig, verbesserte Emitter besonders im Bezug auf Device- und Sublimationsstabilität zur Verfügung zu haben.

Die Verwendung von Indenofluorenen und Indenofluorenderivaten als Materialien in OLEDs wird in JP2000003790 und Le Berre et al., Ann. Chim, 1957, Vol 2, S. 371-425 beschrieben.

In Dahlmann et al., Helvetica Chimica Acta, 1997, 80(1), S. 111-120 wird die Synthese von Bithiophen-Derivaten als funktionelle Materialien zur Verwendung in OLEDs offenbart.

In phosphoreszierenden OLEDs wird als Matrixmaterial häufig 4,4'-Bis-(N-carbazolyl)biphenyl (CBP) verwendet. Die Nachteile sind unter anderem kurze Lebensdauern der mit ihnen hergestellten Devices und häufig hohe Betriebsspannungen, die zu geringen Leistungseffizienzen führen. Außerdem weist CBP eine nicht ausreichend hohe Glasübergangstemperatur auf. Des Weiteren hat sich gezeigt, dass CBP für blau emittierende Elektrolumineszenzvorrichtungen ungeeignet ist, was in einer schlechten Effizienz resultiert. Außerdem ist der Aufbau der Devices komplex, wenn CBP als Matrixmaterial verwendet wird, da zusätzlich eine Lochblockierschicht und eine Elektronentransportschicht verwendet werden müssen. Verbesserte Triplett-Matrixmaterialien, basierend auf Ketoverbindungen von Spirobifluoren, sind in WO 04/093207 beschrieben. Für die besten der dort beschriebenen Matrixmaterialien werden jedoch in der Synthese giftige anorganische Cyanide benötigt, so dass die Herstellung dieser Materialien ökologisch bedenklich ist. Von anderen der dort beschriebenen Matrixmaterialien ist die Glasübergangstemperatur noch nicht zufriedenstellend.

Als Elektronentransportverbindung in organischen Elektrolumineszenzvorrichtungen wird meist AlQ₃ (Aluminium-tris-hydroxychinolinat) verwendet (US 4539507). Dieses hat mehrere Nachteile: Es lässt sich nicht rückstandsfrei aufdampfen, da es sich bei der Sublimationstemperatur teilweise zersetzt, was insbesondere für Produktionsanlagen ein großes Problem darstellt. Ein weiterer Nachteil ist die starke Hygroskopie von AlQ₃, ebenso wie die niedrige Elektronenbeweglichkeit, was zu höheren Spannungen und damit zu einer niedrigeren Leistungseffizienz führt. Um Kurzschlüsse im Display zu vermeiden, würde man gern die Schichtdicke erhöhen; dies ist mit AlQ₃ wegen der geringen Ladungsträgerbeweglichkeit und der daraus resultierenden Spannungserhöhung nicht möglich. Als sehr ungünstig erweist sich weiterhin die Eigenfarbe von AlQ₃ (im Feststoff gelb), die gerade bei blauen OLEDs durch Reabsorption und schwache Reemission zu Farbverschiebungen führen kann. Hier sind blaue OLEDs nur mit starken Effizienz- bzw. Farborteinbußen darstellbar. Trotz der genannten Nachteile stellt AlQ₃ in OLEDs bislang immer noch den besten Kompromiss für die verschiedenartigen Anforderungen an ein Elektronentransportmaterial in OLEDs dar.

Es besteht also weiterhin Bedarf an verbesserten Materialien, insbesondere Host-Materialien für blau fluoreszierende Emitter und Host-Materialien für Triplett-Emitter, aber auch an Emittern, Lochtransportmaterialien und Elektronentransportmaterialien, die in organischen elektronischen Vorrichtungen zu guten Effizienzen und gleichzeitig zu hohen Lebensdauern führen, die bei der Herstellung und beim Betrieb der Vorrichtung zu reproduzierbaren Ergebnissen führen und die synthetisch einfach zugänglich sind.

Überraschend wurde gefunden, dass Verbindungen, die neue, unten beschriebene Struktureinheiten enthalten, deutliche Verbesserungen gegenüber dem Stand der Technik aufweisen. Mit diesen Materialien ist eine Steigerung der Effizienz und der Lebensdauer der organischen elektronischen Vorrichtung im Vergleich zu Materialien gemäß dem Stand der Technik möglich. Weiterhin sind diese Materialien sehr gut für die Verwendung in organischen elektronischen Vorrichtungen geeignet, da sie eine hohe Glasübergangstemperatur aufweisen. Diese Materialien und deren Verwendung in organischen elektronischen Vorrichtungen sind daher der Gegenstand der vorliegenden Erfindung.

Gegenstand der Erfindung sind Verbindungen gemäß Anspruch 1.

Bevorzugt weist die Verbindung gemäß Formel (2a), (3a) und (4a) eine Glasübergangstemperatur T_{g} von größer als 70 °C auf, besonders bevorzugt größer als 100 °C, ganz besonders bevorzugt größer als 130 °C.

Eine Arylgruppe im Sinne dieser Erfindung enthält 6 bis 16 C-Atome; eine Heteroarylgruppe im Sinne dieser Erfindung enthält 2 bis 16 C-Atome und mindestens 1 Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe im Sinne der unten folgenden Definition verstanden.

Ein aromatisches Ringsystem im Sinne dieser Erfindung enthält 6 bis 30 C-Atome im Ringsystem. Ein heteroaromatisches Ringsystem im Sinne dieser Erfindung enthält 2 bis 30 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (bevorzugt weniger als 10 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, etc. als aromatische Ringsysteme im Sinne dieser Erfindung verstanden werden. Dabei kann ein Teil des aromatischen oder heteroaromatischen Ringsystems auch eine kondensierte Gruppe im Sinne der folgenden Definition sein.

Unter einer kondensierten Arylgruppe im Sinne dieser Erfindung wird ein Ringsystem mit 10 bis 16 aromatischen Ringatomen verstanden, in dem mindestens zwei aromatische Ringe miteinander "verschmolzen", d. h. durch Anellierung einander ankondensiert sind, also mindestens eine gemeinsame Kante und ein gemeinsames aromatisches π-Elektronensystem aufweisen. Unter einer kondensierten Heteroarylgruppe im Sinne dieser Erfindung wird ein Ringsystem mit 8 bis 40 aromatischen Ringatomen verstanden, in dem mindestens zwei aromatische oder heteroaromatische Ringe, von denen mindestens einer heteroaromatisch ist, einander ankondensiert sind. Diese Ringsysteme können substituiert oder unsubstituiert sein. Beispiele für kondensierte Aryl- oder Heteroarylgruppen sind Naphthalin, Chinolin, Benzothiophen, Anthracen, Phenanthren, Phenanthrolin, Pyren, Acridin, etc., während beispielsweise Biphenyl keine kondensierte Arylgruppe darstellt, da dort keine gemeinsame Kante zwischen den beiden Ringsystemen vorliegt. Auch beispielsweise Fluoren stellt kein kondensiertes aromatisches Ringsystem dar, da die beiden Phenyl-Einheiten dort kein gemeinsames aromatisches Ringsystem ausbilden.

Im Rahmen der vorliegenden Erfindung werden unter einer C₁- bis Csgeradkettigen oder verzweigten Alkylgruppe, in der auch ein oder mehrere H-Atome durch F ersetzt sein können, oder ein oder mehrere nicht benachbarte CH₂-Gruppen durch durch -R²C=CR²-, -C≡C- oder -O- ersetzt sein können, besonders bevorzugt
die Reste Methyl, Ethyl, n-Propyl, i-Propyl, n-Butyl, i-Butyl, s-Butyl, t-Butyl, 2-Methylbutyl, n-Pentyl, s-Pentyl, Trifluormethyl, Pentafluorethyl, 2,2,2-Trifluorethyl, Ethenyl, Propenyl, Butenyl, Pentenyl, Hexenyl, Ethinyl, Propinyl, Butinyl, Pentinyl, Hexinyl verstanden. Unter einem aromatischen oder heteroaromatischen Ringsystem mit 5 - 30 aromatischen Ringatomen, welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Anspruchsgemäß sind Strukturen gemäß der Formel (2a) (3a) oder (4a), 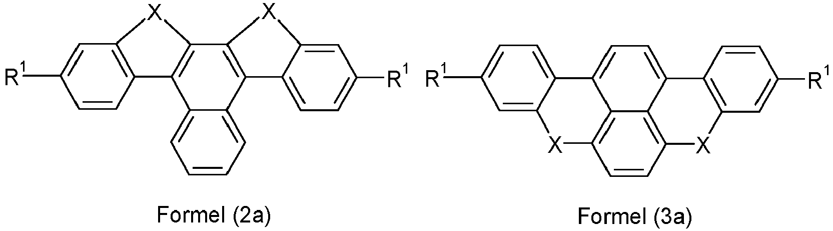 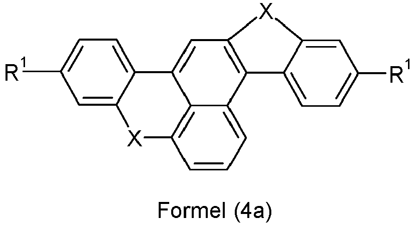 wobei die Symbole X und R¹ dieselbe Bedeutung haben, wie in Anspruch 1 beschrieben, und die Naphthalingruppe durch einen oder mehrere Reste R¹ substituiert sein kann.

Besonders bevorzugt sind in den Strukturen gemäß Formel (2a), (3a) und (4a) beide Reste R¹ ungleich Wasserstoff. Weiterhin bevorzugt ist in diesen Strukturen die zentrale Naphthalingruppe unsubstituiert.

In Verbindungen gemäß Formel (2a), (3a) bzw. (4a) steht R¹ gleich oder verschieden bei jedem Auftreten für H, F, C(=O)Ar, P(=O)(Ar)₂, CR²=CR²Ar, eine geradkettige Alkylgruppe mit 1 bis 5 C-Atomen oder verzweigte Alkylgruppe mit 3 bis 5 C-Atomen, wobei eine oder mehrere nicht benachbarte CH₂-Gruppen durch -R²C=CR²-, -C=C- oder -O- ersetzt sein können und wobei ein oder mehrere H-Atome durch F ersetzt sein können, oder eine Arylgruppe mit 6 bis 16 C-Atomen oder Heteroarylgruppe mit 2 bis 16 C-Atomen oder eine Spirobifluorengruppe, die jeweils mit einem oder mehreren Resten R² substituiert sein kann, oder für eine Kombination aus zwei oder drei dieser Systeme.

Bevorzugt sind in den Strukturen (2a), (3a) und (4a) die Substituenten R¹ gleich gewählt.

Beispiele für Verbindungen gemäß Formel (2a), (3a) und (4a) sind die im Folgenden abgebildeten Strukturen (1) bis (56). Nicht anspruchsgemäß: mit * markiert.

| | |
|---|---|
| 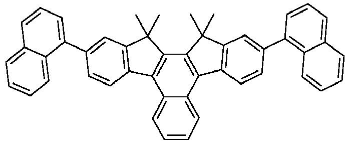 | 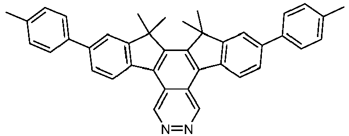 |
| (1)* | (2)* |
| 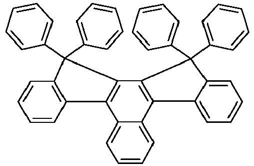 | 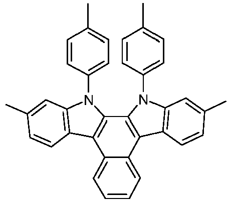 |
| (3)* | (4)* |
| 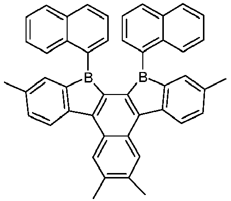 | 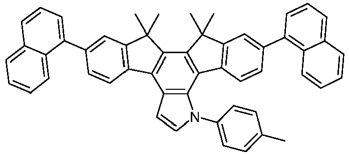 |
| (5)* | (6)* |
| 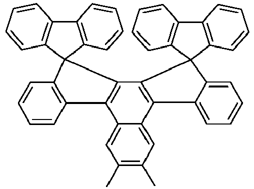 | 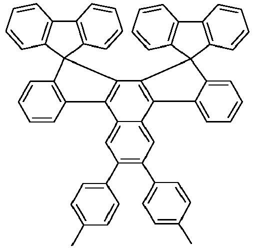 |
| (7)* | (8)* |
| 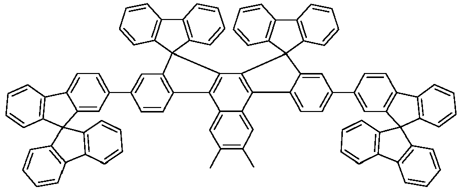 | 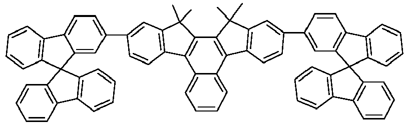 |
| (9)* | (10)* |
| 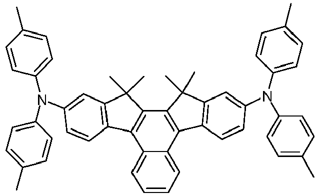 | 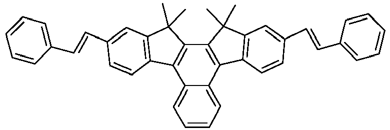 |
| (11)* | (12)* |
| 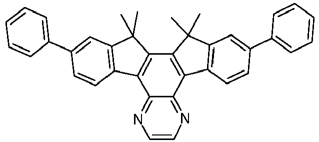 | 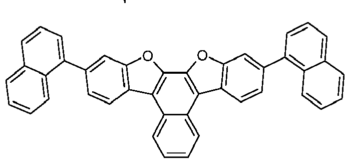 |
| (13)* | (14)* |
| 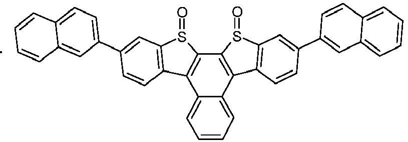 | 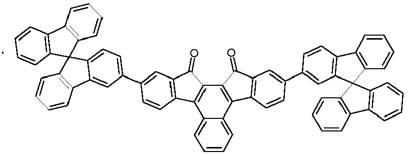 |
| (1b)* | (16)* |
| 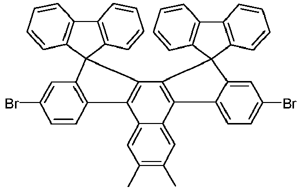 | 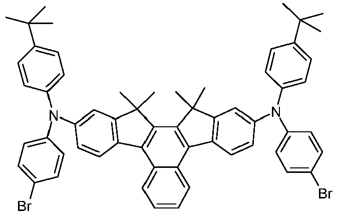 |
| (17)* | (18)* |
| 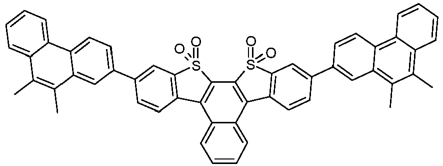 | 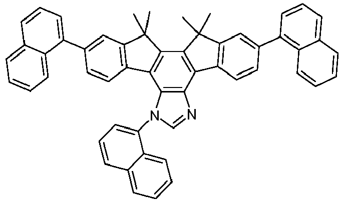 |
| (19)* | (20)* |
| 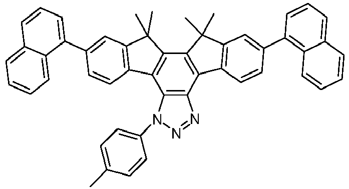 | 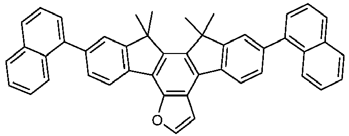 |
| (21)* | (22)* |
| 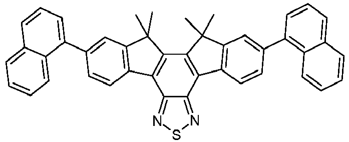 | 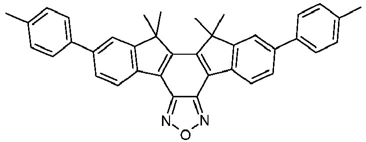 |
| (23)* | (24)* |
| 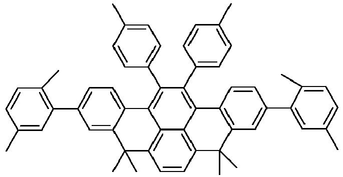 | 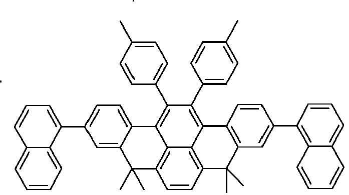 |
| (25)* | (26)* |
| 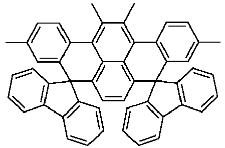 | 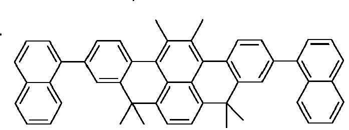 |
| (27)* | (28)* |
| 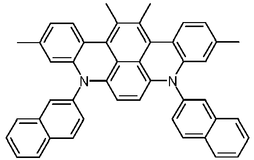 | 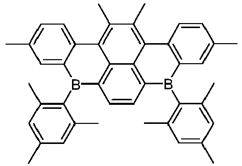 |
| (29)* | (30)* |
| 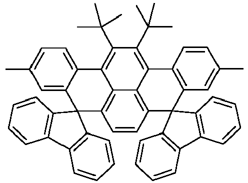 | 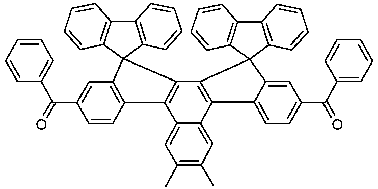 |
| (31)* | (32)* |
| 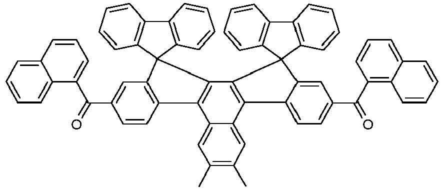 | 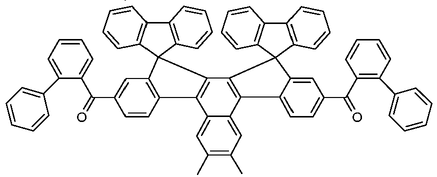 |
| (33)* | (34)* |
| 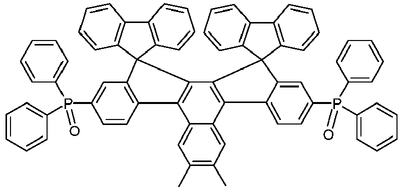 | 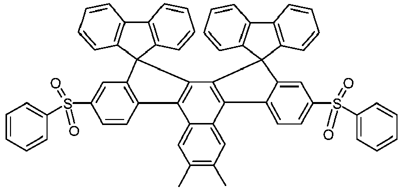 |
| (35)* | (36)* |
| 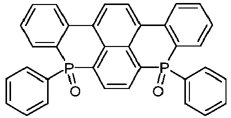 | 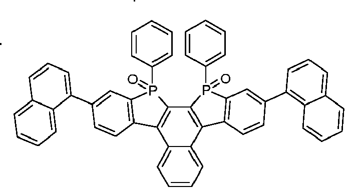 |
| (37)* | (38)* |
| 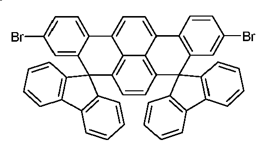 | 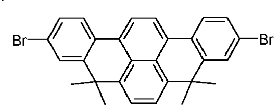 |
| (39)* | (40)* |
| 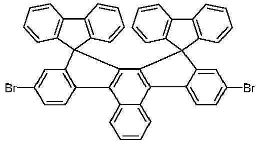 | 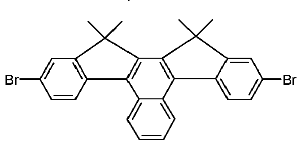 |
| (41)* | (42)* |
| 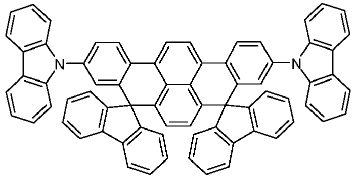 | 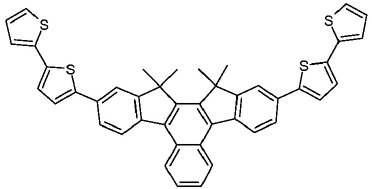 |
| (43)* | (44)* |
| 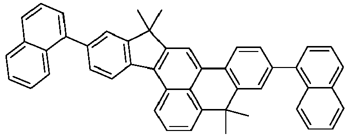 | 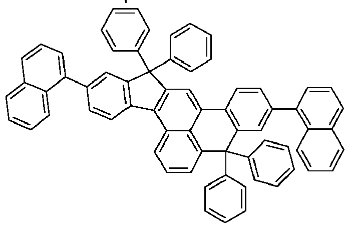 |
| (45)* | (46)* |
| 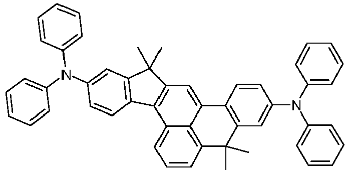 | 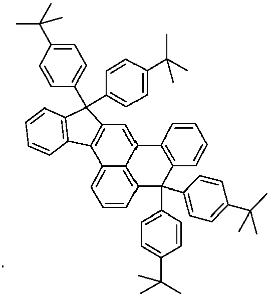 |
| (47)* | (48)* |
| 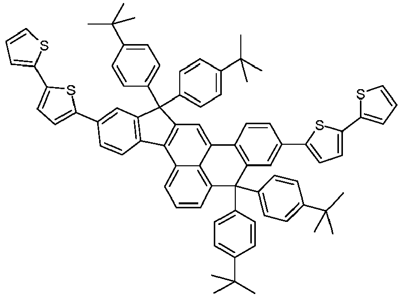 | 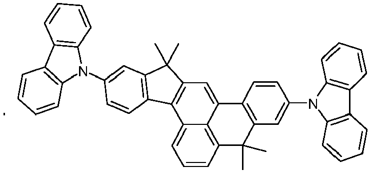 |
| (49)* | (50)* |
| 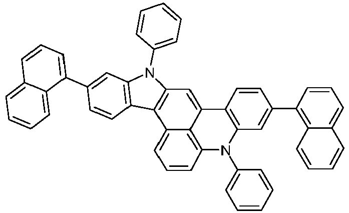 | 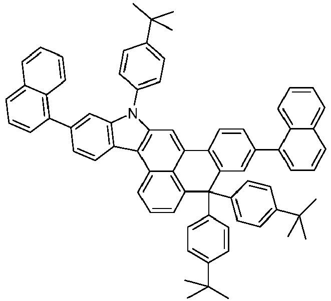 |
| (51)* | (52) |
| 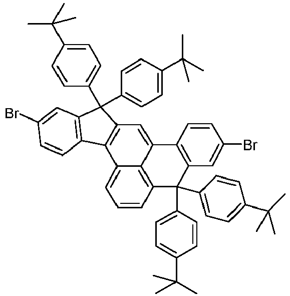 | 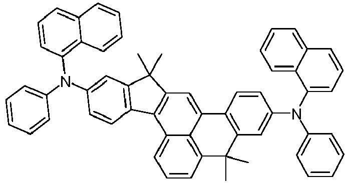 |
| (53)* | (54)* |
| 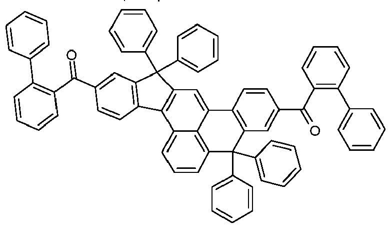 | 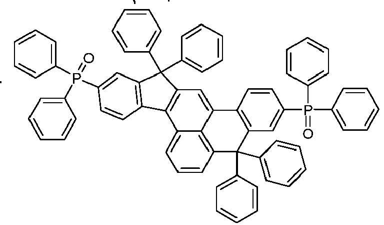 |
| (55)* | (56)* |

Die oben beschriebenen erfindungsgemäßen Verbindungen, insbesondere Verbindungen, welche mit reaktiven Abgangsgruppen wie Brom, Iod, Boronsäure oder Boronsäureester substituiert sind, können auch als Comonomere zur Erzeugung entsprechender konjugierter, teilkonjugierter oder nicht-konjugierter Polymere, Oligomere oder auch als Kern von Dendrimeren Verwendung finden. Diese sind nicht anspruchsgemäß. Die Polymerisation erfolgt dabei bevorzugt über die Halogenfunktionalität bzw. die Boronsäurefunktionalität.

Hierin offenbart, jedoch nicht anspruchsgemäß sind somit konjugierte, teilkonjugierte und nicht-konjugierte Polymere, Oligomere oder Dendrimere enthaltend eine oder mehrere Verbindungen gemäß Formel (2a), (3a) und (4a), wobei ein oder mehrere Reste R¹ Bindungen der Verbindung gemäß Formel (2a), (3a) und (4a) zum Polymer oder Dendrimer darstellen. Für die Wiederholeinheiten gemäß Formel (2a), (3a) und (4a) in Polymeren, Oligomeren und Dendrimeren gelten dieselben Bevorzugungen wie oben beschrieben.

Die Wiederholeinheiten gemäß Formel (2a), (3a) und (4a) werden bevorzugt mit weiteren Comonomeren copolymerisiert. Geeignete und bevorzugte Comonomere sind gewählt aus Fluorenen (z. B. gemäß EP 842208 oder WO 00/22026), Spirobifluorenen, (z. B. gemäß EP 707020, EP 894107 oder EP 04028865.6), Para-phenylenen (z. B. gemäß WO 92/18552), Carbazolen (z. B. gemäß WO 04/070772 oder WO 04/113468), Thiophenen (z. B. gemäß EP 1028136), Dihydrophenanthrenen (z. B. gemäß WO 05/014689), cis- und trans-Indenofluorenen (z. B. gemäß WO 04/041901 oder WO 04/113412), Ketonen (z. B. gemäß WO 05/040302), Phenanthrenen (z. B. gemäß WO 05/104264 oder der nicht offen gelegten Anmeldung DE 102005037734.3) oder auch mehreren dieser Einheiten. Diese Polymere enthalten üblicherweise noch weitere Einheiten, beispielsweise emittierende (fluoreszierende oder phosphoreszierende) Einheiten, wie z. B. Vinyltriarylamine (z. B. gemäß der nicht offen gelegten Anmeldung DE 102005060473.0) oder phosphoreszierende Metallkomplexe (z. B. gemäß WO 06/003000), und/oder Ladungstransporteinheiten, insbesondere solche basierend auf Triarylaminen.

Die erfindungsgemäßen Verbindungen können nach dem Fachmann bekannten Syntheseschritten, wie z. B. Bromierung, Suzuki-Kupplung, Hartwig-Buchwald-Kupplung, Acylierung, Alkylierung, oxidative Cyclisierung, etc., dargestellt werden.

Verbindungen mit Benzo[rst]pentaphen-5,8-dion-Grundgerüst (nicht anspruchsgemäß) können beispielsweise durch oxidative Cyclisierung von 1,4-Dibenzoylnaphthalin in einer eutektischen Aluminiumchlorid-Natriumchlorid-Schmelze dargestellt werden (Schema 1).

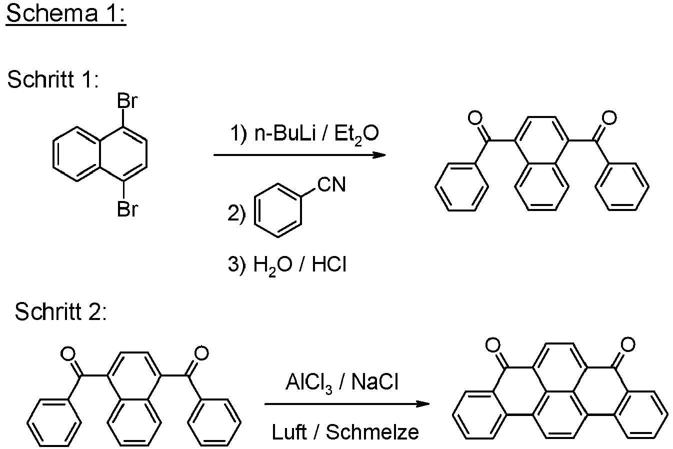

Die anschließende Reduktion des so erhaltenen Benzo[rst]pentaphen-5,8-dion-Grundgerüsts mit Hydrazinhydrat nach Wolf-Kishner bzw. Huang-Minlon, gefolgt von einer Methylierung und einer abschließenden Bromierung, ergibt das Synthon 3,10-Dibrom-5,5,8,8-tetramethyl-benzo[rst]pentaphen (Schema 2).

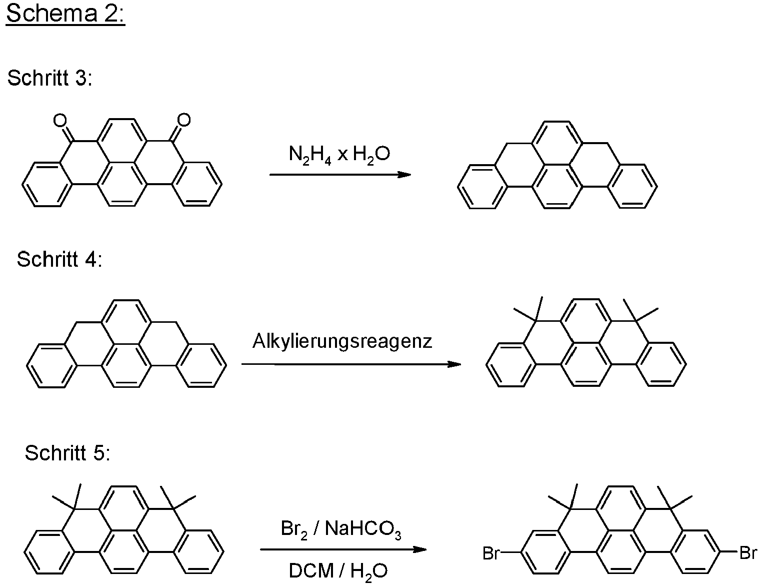

3,10-Dibrom-5,5,8,8-tetramethyl-benzo[rst]pentaphen kann dann z. B. durch Suzuki-Kupplung mit Arylboronsäuren und Arylboronsäurederivaten zu erweiterten aromatischen Kohlenwasserstoffen, durch Buchwald-Kupplung mit Diarylaminen zu Triarylamin-Derivaten oder via Lithiierung und Reaktion mit Elektrophilen wie Benzonitril und anschließender saurer Hydrolyse zu Ketonen oder mit Chlordiphenylphosphinen und anschließender Oxidation zu Phosphinoxiden umgesetzt werden (Schema 3).

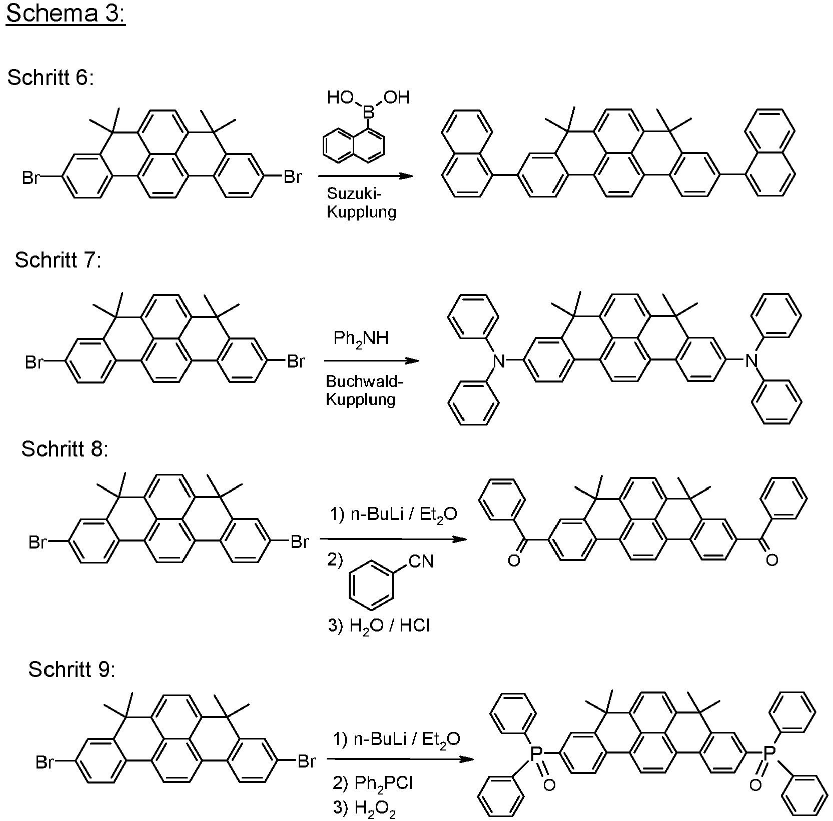

Selbstverständlich kann die Bromierung auch am Benzo[rst]pentaphen-5,8-dion-Grundgerüst erfolgen. Eine anschließende Umsetzung mit Biphenyl-2-magnesiumbromid und eine säurekatalysierte Cyclisierung des intermediären Triarylmethanols ergibt die entsprechende Spiro-Verbindung des Benzo[rst]pentaphens (Schema 4) (nicht anspruchsgemäß), welche wie in Schema 3 beschrieben weiter funktionalisiert werden kann.

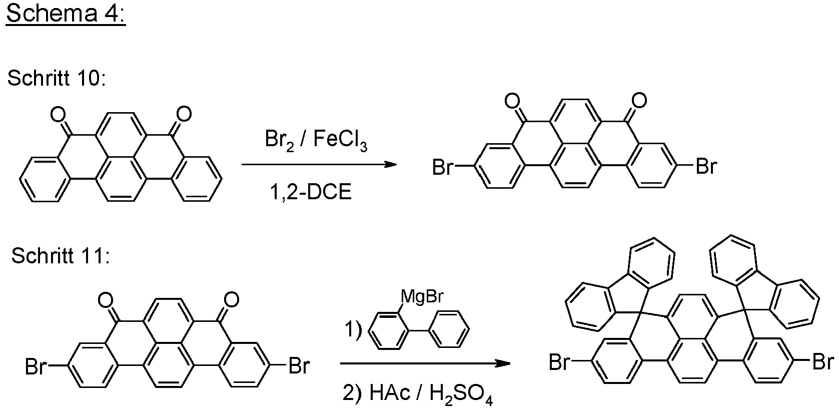

Benz[c]indeno[2,1-a]fluoren-13,14-dion kann gemäß Bulletin Chem. Soc. Jpn. 1977, 50(1), 273 dargestellt werden. Die Bromierung ist mit elementarem Brom in Gegenwart von wasserfreiem Eisen(III)chlorid möglich (analog Schema 2). Das so gewonnene 2,11-Dibrombenz[c]-indeno[2,1-a]fluoren-13,14-dion kann analog den Schemata 2, 3 und 4 weiter funktionalisiert werden.

Ein alternativer Aufbau der Grundgerüste ist ausgehend von 1,4-Bis(2-methoxycarbonylphenyl)naphthalin (nicht anspruchsgemäß) möglich, wie in Schema 5 dargestellt. Dieses wird mit einem Organometallreagenz zum entsprechenden tertiären Alkohol umgesetzt, der säurekatalysiert cyclisiert wird. Das Verhältnis der gebildeten Isomere hängt dabei von den Substituenten und den genauen Synthesebedingungen ab. Die weitere Funktionalisierung kann wie oben beschrieben erfolgen.

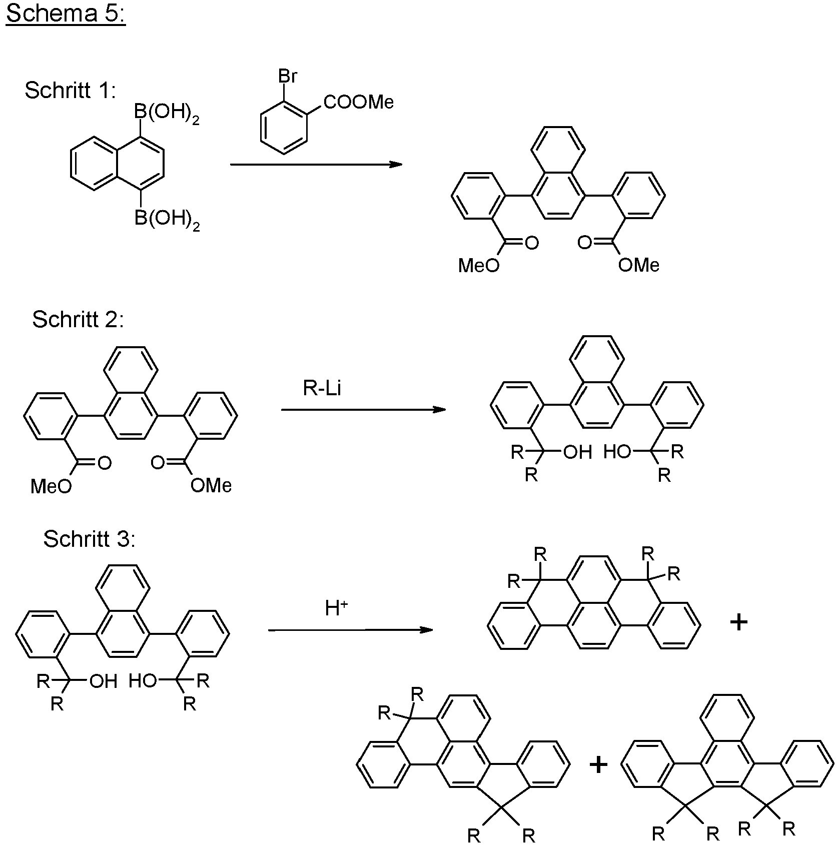

Weiterhin können die bromierten Verbindungen entweder direkt oder nach Überführung in ein Boronsäurederivat als Monomere für Polymere, Oligomere oder Dendrimere eingesetzt werden.

Bei der Synthese können, je nach Synthesebedingungen, sowohl die 5-Ring/5-Ring-Derivate wie auch die 6-Ring/6-Ring-Derivate, die 5-Ring/6-Ring-Derivate oder Mischungen dieser Verbindungen entstehen. Diese können entweder getrennt und als Reinverbindungen weiterverarbeitet werden, oder sie können auch als Mischung eingesetzt werden.

Die Verbindungen gemäß Formel (2a), (3a) und (4a) sind sehr gut für den Einsatz in organischen Elektrolumineszenzvorrichtungen (OLEDs, PLEDs) geeignet. Abhängig von der Substitution wird die Verbindung in unterschiedlichen Funktionen in der OLED eingesetzt.

Ein weiterer Gegenstand der Erfindung ist daher die Verwendung von Verbindungen gemäß Formel (2a), (3a) und (4a) in organischen elektronischen Vorrichtungen, insbesondere in organischen Elektrolum ineszenzvorrichtungen.

Nochmals ein weiterer Gegenstand der Erfindung sind organische elektronische Vorrichtungen, enthaltend mindestens eine Verbindung gemäß Formel (2a), (3a) und (4a), insbesondere organische Elektrolumineszenzvorrichtungen, enthaltend Anode, Kathode und mindestens eine emittierende Schicht, dadurch gekennzeichnet, dass mindestens eine organische Schicht mindestens eine Verbindung gemäß Formel (2a), (3a) und (4a) enthält.

Außer Kathode, Anode und der emittierenden Schicht kann die organische Elektrolumineszenzvorrichtung noch weitere Schichten enthalten. Diese können beispielsweise sein: Lochinjektionsschicht, Lochtransportschicht, Elektronentransportschicht, Elektroneninjektionsschicht und/oder Charge-Generation Layer (IDMC 2003, Taiwan; Session 21 OLED (5), T. Matsumoto, T. Nakada, J. Endo, K. Mori, N. Kawamura, A. Yokoi, J. Kido, Mu/tiphoton Organic EL Device Having Charge Generation Layer). Es sei aber darauf hingewiesen, dass nicht notwendigerweise jede dieser Schichten vorhanden sein muss.

In einer weiteren bevorzugten Ausführungsform der Erfindung enthält die organische Elektrolumineszenzvorrichtung mehrere emittierende Schichten, wobei mindestens eine Schicht mindestens eine Verbindung gemäß Formel (2a), (3a) und (4a) enthält. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die gelbes, orange oder rotes Licht emittieren. Insbesondere bevorzugt sind Dreischichtsysteme, wobei mindestens eine dieser Schichten mindestens eine Verbindung gemäß Formel (2a), (3a) und (4a) enthält und wobei die drei Schichten blaue, grüne und orange oder rote Emission zeigen (für den prinzipiellen Aufbau siehe z. B. WO 05/011013). Ebenso eignen sich für weiße Emission Emitter, welche breitbandige Emissionsbanden aufweisen und dadurch weiße Emission zeigen.

Bei Verwendung der Verbindungen gemäß Formel (2a), (3a) und (4a) als Host für einen fluoreszierenden Dotanden sind bevorzugt ein oder mehrere Substituenten R¹ gewählt aus einfachen oder kondensierten Aryl- oder Heteroarylgruppen, insbesondere Phenyl, ortho-, meta- oder para-Biphenyl, 1- oder 2-Naphthyl, Anthryl, insbesondere Phenylanthryl oder 1- oder 2-Naphthylanthryl, 2-Fluorenyl und 2-Spirobifluorenyl, welche jeweils durch einen oder mehrere Reste R² substituiert sein können.

Unter einem Hostmaterial wird in einem System aus Host und Dotand diejenige Komponente verstanden, die in dem System im höheren Anteil vorliegt. Bei einem System aus einem Host und mehreren Dotanden wird als Host diejenige Komponente verstanden, deren Anteil der höchste in der Mischung ist.

Der Anteil des Hostmaterials gemäß der Formel (2a), (3a) und (4a) in der emittierenden Schicht beträgt zwischen 50.0 und 99.9 Gew.-%, bevorzugt zwischen 80.0 und 99.5 Gew.-%, besonders bevorzugt zwischen 90.0 und 99.0 Gew.-%. Entsprechend beträgt der Anteil des Dotanden zwischen 0.1 und 50.0 Gew.-%, bevorzugt zwischen 0.5 und 20.0 Gew.-%, besonders bevorzugt zwischen 1.0 und 10.0 Gew.-%.

In fluoreszierenden Vorrichtungen ist der Dotand bevorzugt ausgewählt aus der Klasse der Monostyrylamine, der Distyrylamine, der Tristyrylamine, der Tetrastyrylamine und der Arylamine. Unter einem Monostyrylamin wird eine Verbindung verstanden, die eine Styrylgruppe und mindestens ein Amin enthält, welches bevorzugt aromatisch ist. Unter einem Distyrylamin wird eine Verbindung verstanden, die zwei Styrylgruppen und mindestens ein Amin enthält, welches bevorzugt aromatisch ist. Unter einem Tristyrylamin wird eine Verbindung verstanden, die drei Styrylgruppen und mindestens ein Amin enthält, welches bevorzugt aromatisch ist. Unter einem Tetrastyrylamin wird eine Verbindung verstanden, die vier Styrylgruppen und mindestens ein Amin enthält, welches bevorzugt aromatisch ist. Unter einem Arylamin bzw. einem aromatischen Amin im Sinne dieser Erfindung wird eine Verbindung verstanden, die drei aromatische oder heteroaromatische Ringsysteme direkt an den Stickstoff gebunden enthält, davon bevorzugt mindestens ein kondensiertes Ringsystem mit mindestens 14 aromatischen Ringatomen. Die Styrylgruppen sind besonders bevorzugt Stilbene, die auch an der Doppelbindung oder an den Aromaten weiter substituiert sein können. Beispiele für derartige Dotanden sind substituierte oder unsubstituierte Tristilbenamine oder weitere Dotanden, die beispielsweise in WO 06/000388 und in den nicht offen gelegten Patentanmeldungen DE 102004031000.9, EP 04028407.7 und EP 05001891.0 beschrieben sind. Außerdem sind Verbindungen gemäß DE 102005023437.2 bevorzugt.

Bei Verwendung der Verbindungen gemäß Formel (2a), (3a) und (4a) als Host für phosphoreszierende Dotanden enthalten bevorzugt ein oder mehrere Substituenten R¹ mindestens eine Gruppe C=O und/oder P(=O)(R²). Besonders bevorzugt sind diese Gruppen direkt an die erfindungsgemäße zentrale Einheit gebunden und enthalten weiterhin besonders bevorzugt noch einen bzw. im Fall des Phosphinoxids zwei weitere aromatische Substituenten.

In phosphoreszierenden Vorrichtungen ist der Dotand bevorzugt ausgewählt aus der Klasse der Metallkomplexe, enthaltend mindestens ein Element der Ordnungszahl größer 20, bevorzugt größer 38 und kleiner 84, besonders bevorzugt größer 56 und kleiner 80. Bevorzugt werden als Phosphoreszenzemitter Metallkomplexe verwendet, die Molybdän, Wolfram, Rhenium, Ruthenium, Osmium, Rhodium, Iridium, Palladium, Platin, Silber, Gold oder Europium enthalten, insbesondere Iridium oder Platin. Generell eignen sich hierfür phosphoreszierende Materialien, wie sie gemäß dem Stand der Technik verwendet werden.

Es ist weiterhin bevorzugt, wenn die Verbindungen gemäß Formel (2a), (3a) und (4a) als emittierende Materialien eingesetzt werden, wenn mindestens ein Substituent R¹ mindestens eine Vinylaryl-Einheit, und/oder mindestens eine Stilben-Einheit enthält.

Der Anteil der Verbindung gemäß Formel (2a), (3a) und (4a) in der Mischung der emittierenden Schicht zwischen 0.1 und 50.0 Gew.-%, bevorzugt zwischen 0.5 und 20.0 Gew.-%, besonders bevorzugt zwischen 1.0 und 10.0 Gew.-%. Entsprechend beträgt der Anteil des Hostmaterials zwischen 50.0 und 99.9 Gew.-%, bevorzugt zwischen 80.0 und 99.5 Gew.-%, besonders bevorzugt zwischen 90.0 und 99.0 Gew.-%.

Als Hostmaterialien kommen dann verschiedene Stoffklassen in Frage. Bevorzugte Hostmaterialien sind ausgewählt aus den Klassen der Oligo-arylene (z. B. 2,2',7,7'-tetraphenyl-spirobifluoren gemäß EP 676461 oder Dinaphthylanthracen), insbesondere der Oligo-arylene enthaltend kondensierte aromatische Gruppen, der Oligo-arylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911), der elektronenleitenden Verbindungen, insbesondere Ketone, Phosphinoxide, Sulfoxide, etc. (z. B. gemäß WO 05/084081 und WO 05/084082), der Atropisomere (z. B. gemäß der nicht offen gelegten Anmeldung EP 04026402.0) oder der Boronsäurederivate (z. B. gemäß der nicht offen gelegten Anmeldung EP 05009643.7). Weiterhin kommen als Hostmaterialien die oben beschriebenen erfindungsgemäßen Verbindungen in Frage. Besonders bevorzugte Hostmaterialien sind außer den erfindungsgemäßen Verbindungen ausgewählt aus den Klassen der Oligoarylene, enthaltend Naphthalin, Anthracen und/oder Pyren oder Atropisomeren dieser Verbindungen, der Oligo-arylenvinylene, der Ketone, der Phosphinoxide und der Sulfoxide. Ganz besonders bevorzugte Hostmaterialien sind außer den erfindungsgemäßen Verbindungen ausgewählt aus den Klassen der Oligoarylene, enthaltend Anthracen und/oder Pyren oder Atropisomeren dieser Verbindungen, der Phosphinoxide und der Sulfoxide. Unter einem Oligoarylen im Sinne dieser Erfindung soll eine Verbindung verstanden werden, in der mindestens drei Aryl- bzw. Arylengruppen aneinander gebunden sind.

In nochmals einer weiteren Ausführungsform der Erfindung werden die Verbindungen gemäß Formel (2a), (3a) und (4a) als Lochtransportmaterial bzw. als Lochinjektionsmaterial eingesetzt. Die Verbindung wird bevorzugt in einer Lochtransport- bzw. in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen einer Lochinjektionsschicht und einer Emissionsschicht liegt. Wenn die Verbindungen gemäß Formel (2a), (3a) und (4a) als Lochtransport- bzw. als Lochinjektionsmaterial verwendet werden, kann es bevorzugt sein, wenn sie mit Elektronenakzeptor-Verbindungen dotiert werden, beispielsweise mit F₄-TCNQ oder mit Verbindungen, wie in EP 1476881 oder EP 1596445 beschrieben.

Es ist weiterhin bevorzugt, wenn die Verbindungen gemäß Formel (2a), (3a) und (4a) als Elektronentransportmaterial eingesetzt werden. Hier kann es bevorzugt sein, wenn ein oder mehrere Substituenten R¹ mindestens eine Gruppe C=O und/oder P(=O)(R²) enthalten. Besonders bevorzugt sind diese Gruppen direkt an die erfindungsgemäße zentrale Einheit gebunden und enthalten weiterhin besonders bevorzugt noch einen bzw. im Fall des Phosphinoxids zwei weitere aromatische Substituenten. Weiterhin kann es bevorzugt sein, wenn die Verbindung mit Elektronendonator-Verbindungen dotiert ist. Weiterhin kann es bevorzugt sein, wenn die Verbindung mit Elektronendonator-Verbindungen dotiert ist.

Auch in Polymeren können Verbindungen gemäß Formel (2a), (3a) und (4a) entweder als Polymergrundgerüst (Backbone), als emittierende Einheit und/oder als lochtransportierende Einheit eingesetzt werden. Dabei entsprechen die bevorzugten Substitutionsmuster den oben beschriebenen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (2a), (3a) und (4a) nötig. Hohe Löslichkeit lässt sich durch geeignete Substitution der Verbindungen erreichen.

Die erfindungsgemäßen Verbindungen weisen bei Verwendung in organischen Elektrolumineszenzvorrichtungen eine hohe Effizienz und eine hohe Stabilität, was sich vor allem in einer hohen Lebensdauer zeigt, auf. Außerdem weisen die Verbindungen eine hohe Glasübergangstemperatur auf.

Im vorliegenden Anmeldetext wird auf die Verwendung der erfindungsgemäßen Verbindungen in Bezug auf OLEDs und PLEDs und die entsprechenden Displays abgezielt. Trotz dieser Beschränkung der Beschreibung ist es für den Fachmann ohne weiteres erfinderisches Zutun möglich, die erfindungsgemäßen Verbindungen auch für weitere Verwendungen in anderen elektronischen Vorrichtungen einzusetzen, z. B. für organische Feld-Effekt-Transistoren (O-FETs), organische Dünnfilm transistoren (O-TFTs), organische lichtemittierende Transistoren (O-LETs), organische integrierte Schaltungen (O-ICs), organische Solarzellen (O-SCs), organische Feld-Quench-Devices (O-FQDs), lichtemittierende elektrochemische Zellen (LECs), organische Laserdioden (O-Laser) oder organischen Photorezeptoren.

Die Verwendung der erfindungsgemäßen Verbindungen in den entsprechenden Vorrichtungen ebenso wie diese Vorrichtungen selbst sind ebenfalls ein Gegenstand der vorliegenden Erfindung.

Die Erfindung wird durch die nachfolgenden Beispiele näher erläutert, ohne sie dadurch einschränken zu wollen.

Beispiele:
Die nachfolgenden Synthesen wurden, sofern nicht anders angegeben, unter einer Schutzgasatmosphäre durchgeführt. Die Edukte wurden von den Firmen ALDRICH bzw. ABCR bezogen. 1,4-Naphthalindiboronsäure wurde gemäß *Journal of Organic Chemistry* **2000,** *65(13)*, 3952-3960 synthetisiert. Nicht anspruchsgemäße Beispiele sind mit * gekennzeichnet.

### Beispiel 1 *: Synthese von 4,10-Tetramethyl-4H-10H-fluoreno[4,3,2-de]anthracen und 5,8-Tetramethyl-5,8-dihydro-benzo[rst]pentaphen

### a) 1,4-Bis(2-methoxycarbonylphenyl)naphthalin

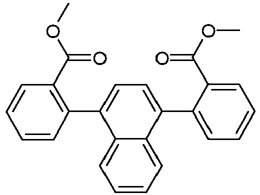

Eine gut gerührte, entgaste Suspension aus 21.6 g (7.1 mmol) 2-Brombenzoesäuremethylester, 10.1 g (28 mmol) 1,4-Naphthalindiboronsäure und 18.9 g (6.6 mmol) Triskaliumphosphat in einem Gemisch aus 350 ml Wasser und 350 ml THF wird mit 1.55 g (0.1 mmol) Pd(PPh₃)₄ versetzt und 60 h unter Rückfluss erhitzt. Nach Erkalten wird die organische Phase abgetrennt, dreimal mit 200 ml Wasser und einmal mit 200 ml gesättigter Kochsalzlösung gewaschen und anschließend über Magnesiumsulfat getrocknet. Die organische Phase wird im Vakuum zur Trockene einrotiert. Der so erhaltene graue Rückstand wird aus Dioxan umkristallisiert. Die ausgefallenen Kristalle werden abgesaugt, mit 50 ml Ethanol gewaschen und anschließend im Vakuum getrocknet. Ausbeute: 13 g, 82 % d. Th.

### b) 1,4-Bis(2-methanol-α,α-dimethyl)phenyl)naphthalin

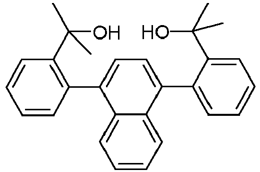

30 g (75 mmol) 1,4-Bis(2-methoxycarbonylphenyl)naphthalin werden in 2000 ml THF unter Stickstoff vorgelegt, auf -78 °C gekühlt und tropfenweise mit 175 ml (378 mmol) 2.2 M Methyllithium-Lösung versetzt. Anschließend wird 16 h bei -78°C gerührt. Man lässt über Nacht auf Raumtemperatur kommen. Nach Hydrolyse mit 125 ml gesättigter NH₄Cl-Lösung wird der Niederschlag abgesaugt und mit Ethylacetat gewaschen. Das Filtrat wird zweimal mit Wasser extrahiert und die organische Phase über Na₂SO₄ getrocknet. Der Rückstand nach Abdampfen des Lösemittels wird chromatographisch gereinigt (Toluol /Ethylacetat 6:4). Ausbeute: 27 g, 93 % d. Th.

### c) 4,10-Tetramethyl-4H-10H-fluoreno[4,3,2-de]anthracen und 5,8-Tetramethyl-5,8-dihydro-benzo[rst]pentaphen

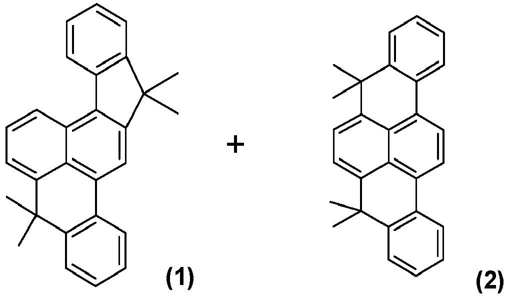

200 g Polyphosphorsäure werden vorgelegt. Anschließend werden 25 g (63.05 mmol) 1,4-Bis(2-methanol-α,α-dimethyl)phenyl)naphthalin zugegeben. Die Mischung wird 20 h bei Raumtemperatur gerührt. Danach wird die Mischung langsam mit Eiswasser versetzt. Der ausgefallene Feststoff wird abgesaugt, im Trockenschrank getrocknet und aus Ethylacetat umkristallisiert.

Ausbeute:
4,10-Tetramethyl-4H-10H-fluoreno[4,3,2-de]anthracen **(1):** 9.9 g (45 % d. Th.)
5,8-Tetramethyl-5,8-dihydro-benzo[rst]pentaphen **(2):** 6.6 g (30 % d. Th.)

### Beispiel 2 *: Synthese von 5,8-Tetra(p-tert-butylphenyl)-5,8-dihydro-benzo[rst]pentaphen und 4,10-Tetra(p-tert-butylphenyl)-4H-10H-fluoreno[4,3,2-de]anthracen

### a) 1,4-Bis[2-methanol-α,α-di(p-tert-butylphenyl)phenyl]naphthalin

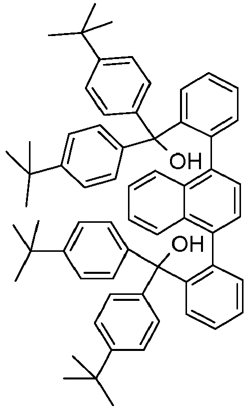

8.5 g (40 mmol) 1-Brom-4-tert-butylbenzol werden in 50 ml THF unter Stickstoff vorgelegt, auf -75 °C gekühlt, tropfenweise mit 25 ml (40 mmol) 1.6 M n-Butyllithium-Lösung versetzt und 2 h bei dieser Temperatur gerührt. Anschließend werden 3.56 g (9 mmol) 1,4-Bis(2-methoxycarbonyl-phenyl)naphthalin (synthetisiert gemäß Beispiel 1a), gelöst in 50 ml THF, so zugetropft, dass die Temperatur -65 °C nicht übersteigt. Man lässt über Nacht auf Raumtemperatur kommen. Nach Hydrolyse mit 200 ml Wasser wird der Niederschlag abgesaugt, mit EtOH nachgewaschen und aus Ethylacetat umkristallisiert. Ausbeute: 15.8 g, 85 % d. Th.

### b) 5,8-Tetra(p-tert-butylphenyl)-5,8-dihydro-benzo[rst]pentaphen und 4,10-Tetra(p-tert-butylphenyl)-4H-10H-fluoreno[4,3,2-de]anthracen

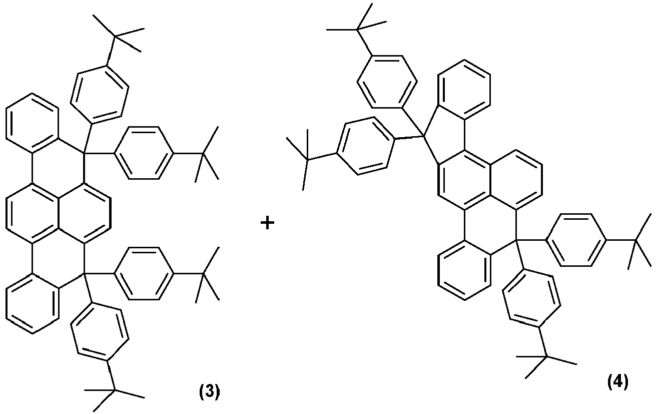

9.7 g (11.2 mmol) 1,4-Bis[2-methanol-α,α-di(p-*tert*-butylphenyl)phenyl]-naphthalin werden in 120 ml Eisessig vorgelegt und 10 min. gerührt. Anschließend werden 0.5 ml HCl (konz.) zugegeben und 1 h unter Rückfluss gekocht. Danach wird die Mischung langsam mit Eiswasser versetzt. Der ausgefallene Feststoff wird abgesaugt, im Trockenschrank getrocknet und aus Toluol umkristallisiert.

Ausbeute:
5,8-Tetra(p-*tert*-butylphenyl)-5,8-dihydro-benzo[rst]pentaphen **(3):** 5.8 g, 50 % d. Th.
4,10-Tetra(p-*tert*-butylphenyl)-4H-10H-fluoreno[4,3,2-de]anthracen **(4):** 2.8 g, 30 % d. Th.

### Beispiel 3 *: Synthese von Amin D1

### a) Bromierung von Verbindung (1)

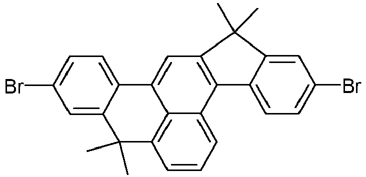

9.0 g (25 mmol) 4,10-Tetramethyl-4H-10H-fluoreno[4,3,2-de]anthracen **(1)** werden unter Lichtausschluss in 300 ml Dichlormethan vorgelegt und auf 5 °C abgekühlt. Es werden 2.7 ml (50 mmol) Brom in 25 ml Dichlormethan innerhalb 15 min. zugetropft und bei 5 °C weitere 7 h gerührt. Nach vollständigem Umsatz wird die Reaktion durch Zugabe von 15 ml Ethanol gestoppt, abgesaugt, mehrfach mit Ethanol gewaschen und anschließend zweimal aus NMP umkristallisiert. Man erhält 11.2 g (86 % d. Th.) eines hellgelben Feststoffs, der gemäß HPLC eine Reinheit von > 99.7 % aufweist.

### b) Synthese von Amin D1

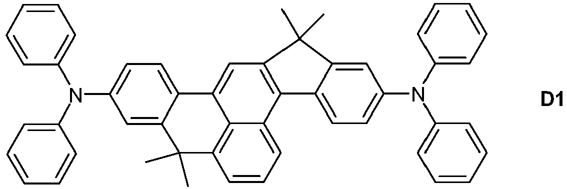

11.2 g (22 mmol) der Bromverbindung aus Beispiel 3a) und 9.7 g (58 mmol) Diphenylamin werden in 250 ml wasserfreiem Toluol suspendiert. Anschließend werden 190 mg (0.9 mmol) Tri-*tert*-butylphosphin sowie 107 mg (0.5 mmol) Pd(OAc)₂ und 6.3 g (66 mmol) NaO^{t}Bu zugegeben, und das Reaktionsgemisch wird 4 h unter Rückfluss erhitzt. Nach beendeter Reaktion werden 150 ml Wasser zugegeben, der Feststoff abgesaugt, mit Ethanol gewaschen und getrocknet. Nach fünfacher Umkristallisation aus NMP, zweimaligem Auskochen mit Ethanol und anschließender zweifacher Sublimation (330 °C, 2 x 10⁻⁵ mbar) erhält man 11.8 g (77 %) eines hellgelben Feststoffs mit einer Reinheit von > 99.9 % gemäß HPLC.

### Beispiele 4 bis 6 *: Synthese der Amine D2, D3 und D4

In Analogie zu Beispiel 3 werden aus den Verbindungen **(2), (3)** und **(4)** (synthetisiert gemäß den Beispielen 1 und 2) durch Bromierung und Hartwig-Buchwald-Kupplung die entsprechenden Bis(diphenylamin)-Derivate synthetisiert. Die Strukturen von **D2, D3** und **D4** sind im Folgenden abgebildet:

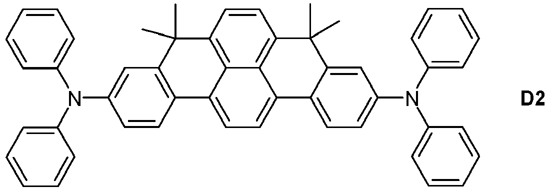

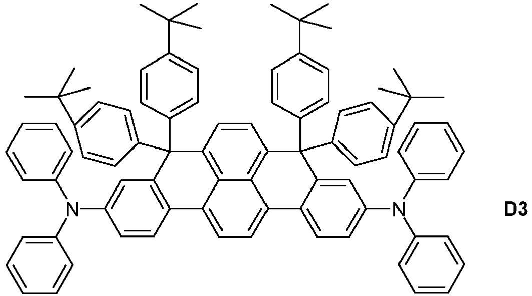

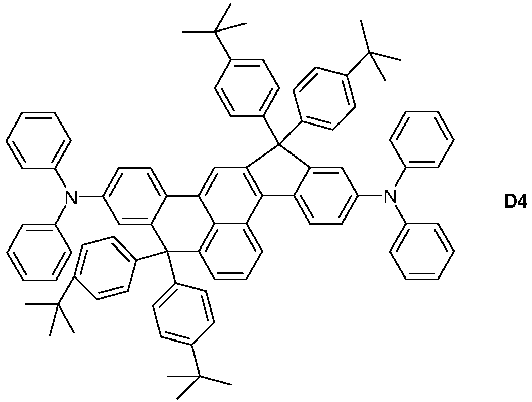

### Beispiel 7 *: Herstellung der OLEDs

Die Herstellung der OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen werden die Ergebnisse verschiedener OLEDs vorgestellt. Der grundlegende Aufbau, die verwendeten Materialien und Schichtdicken, außer der emittierenden Schicht und der Lochinjektionsschicht, sind zur besseren Vergleichbarkeit identisch. Analog dem o. g. allgemeinen Verfahren werden OLEDs mit folgendem Aufbau erzeugt:

| | |
|---|---|
| Lochinjektionsschicht (HIL) | 0 nm oder 20 nm **HIL1,** siehe Tabelle 1 |
| Lochtransportschicht (HTM) | 40 nm oder 20 nm NPB (aufgedampft; N-Naphthyl-N-phenyl-4,4'-diaminobiphenyl) |
| Emissionschicht (EML) | siehe Tabelle 1 für Materialien, Konzentration und Schichtdicke |
| Elektronenleiter (ETL) | 20 nm Alq₃ (bezogen von SynTec; Tris(chinolinato)aluminium(III)) |
| LiF-Al (Kathode) | 1 nm LiF, darauf 150 nm Al. |

Diese OLEDs werden standardmäßig charakterisiert; hierfür werden die Elektrolumineszenzspektren, die Effizienz (gemessen in cd/A), die Leistungseffizienz (gemessen in Im/W) in Abhängigkeit der Helligkeit, berechnet aus Strom-Spannungs-Leuchtdichte-Kennlinien (IUL-Kennlinien), und die Lebensdauer bestimmt. Als Lebensdauer wird die Zeit definiert, nach der die Anfangshelligkeit von 1000 cd/m²auf die Hälfte gesunken ist.

In Tabelle 1 sind die Ergebnisse einiger OLEDs (Beispiele 8 bis 11) zusammengefasst, wobei jeweils die Zusammensetzung der EML und der HTL inklusive der Schichtdicken mit aufgeführt ist. Dabei enthalten die OLEDs die erfindungsgemäßen Verbindungen entweder als Lochinjektionsmaterial (Verwendung von **D3** in Beispiel 9 und 11), als Hostmaterial in der emittierenden Schicht (Verwendung von **(3)** in Beispiel 8 und 9) und/oder als Dotand in der emittierenden Schicht (Verwendung von **D3** in Beispiel 10 und 11). Die Strukturen des Hostmaterials **H1** und des Dotanden **D5** sind im Folgenden dargestellt:

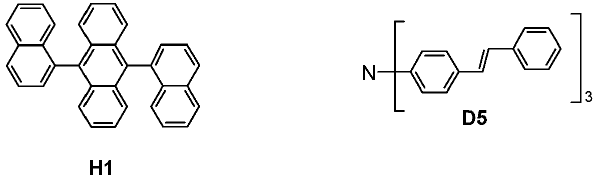

**Tabelle 1**

| Bsp. | HIL | HTL2 | EML | Max. Eff. (cd/A) | U (V) bei 1000 cd/m² | CIE ^{a} | Lebensdauer (h) ^{b} |
|---|---|---|---|---|---|---|---|
| 8* | | **NPB** (40 nm) | **(3):D5 (5%)** (30 nm) | 3.8 | 6.8 | x=0.15 | 700 |
| | | | | | | y=0.11 | |
| 9* | **D3** (20 nm) | **NPB** (20 nm) | **(3):D5 (5%)** (30 nm) | 4.0 | 6.0 | x=0.15 | 2200 |
| | | | | | | y=0.11 | |
| 10* | - | **NPB** (40 nm) | **H1:D3 (5%)** (30 nm) | 9.0 | 5.6 | x=0.18 | 5500 |
| | | | | | | y=0.24 | |
| 11* | **D3** (20 nm) | **NPB** (20 nm) | **H1:D3 (5%)** (30 nm) | 11.0 | 5.8 | x=0.18 | 8000 |
| | | | | | | y=0.24 | |

| | | | | | | | |
|---|---|---|---|---|---|---|---|
| ^{a} CIE-Koordinaten: Farbkoordinaten der Commision Internationale de l'Eclairage 1931. ^{b} Lebensdauer: Zeit bis zum Abfall der Helligkeit auf 50 % der Anfangshelligkeit, gemessen bei einer Anfangshelligkeit von 1000 cd/m². | | | | | | | |

Zusammenfassend kann gesagt werden, dass OLEDs, enthaltend Verbindungen gemäß Formel (2a), (3a) oder (4a), sehr gute Lebensdauern und Effizienzen aufweisen, wie man leicht Tabelle 1 entnehmen kann. Daher eignen sich diese Verbindungen sehr gut für die Verwendung in OLEDs.

## Patentansprüche

1. Verbindungen gemäß Formel (2a), (3a) oder (4a) 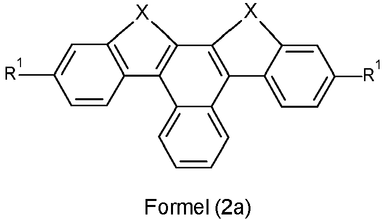 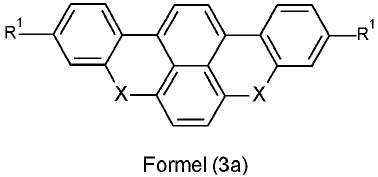 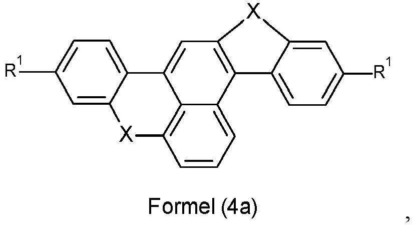 wobei für die verwendeten Symbole und Indizes gilt:
R¹ steht bei jedem Auftreten gleich oder verschieden für H, F, C(=O)Ar, P(=O)(Ar)₂, CR²=CR²Ar, eine geradkettige Alkylgruppe mit 1 bis 5 C-Atomen oder verzweigte Alkylgruppe mit 3 bis 5 C-Atomen, wobei eine oder mehrere nicht benachbarte CH₂-Gruppen
durch -R²C=CR²-, -C=C- oder -O- ersetzt sein können und wobei ein oder mehrere H-Atome durch F ersetzt sein können, oder eine Arylgruppe mit 6 bis 16 C-Atomen oder Heteroarylgruppe mit 2 bis 16 C-Atomen oder eine Spirobifluorengruppe, die jeweils mit einem oder mehreren Resten R² substituiert sein kann, oder eine Kombination aus zwei oder drei dieser Systeme;
Ar ist bei jedem Auftreten ein aromatisches oder heteroaromatisches Ringsystem mit 5-30 aromatischen Ringatomen, das mit einem oder mehreren nicht-aromatischen Resten R¹ substituiert sein kann;
R² ist bei jedem Auftreten gleich oder verschieden H oder ein aliphatischer, aromatischer oder heteroaromatischer Kohlenwasserstoffrest mit 1 bis 20 C-Atomen;
X ist bei jedem Auftreten verschieden eine bivalente Brücke, ausgewählt aus C(R¹)₂, C=O, C=NR¹, O, S, S=O, SO₂, N(R¹), P(R¹), P(=O)R¹, C(R¹)₂-C(R¹)₂, C(R¹)₂-C(R¹)₂-C(R¹)₂, C(R¹)₂-O und C(R¹)₂-O-C(R¹)₂;
wobei die Naphthalingruppe durch einen oder mehrere Reste R¹ substituiert sein kann;
**dadurch gekennzeichnet, dass** mindestens ein Substituent R¹ vorhanden ist, der mindestens eine Aryl- oder Heteroarylgruppe enthält.

2. Verbindungen nach Anspruch 1, **dadurch gekennzeichnet, dass** R¹ bei jedem Auftreten gleich oder verschieden gewählt ist aus H, Arylgruppen mit 6 bis 16 C-Atomen und Heteroarylgruppen mit 2 bis 16 C-Atomen, wobei die Arylgruppen und die Heteroarylgruppen jeweils mit einem oder mehreren Resten R² substituiert sein können.

3. Verbindungen gemäß Anspruch 1 oder 2, **dadurch gekennzeichnet, dass** die in Formel (2a), (3a) und (4a) gezeigten Reste R¹ beide gleich H sind und die Naphthalingruppe nicht durch Reste R¹ substituiert ist, und mindestens ein X in der Formel gewählt aus Formeln (2a), (3a) und (4a) gewählt ist aus C(R¹)₂, C=NR¹, N(R¹), P(R¹), P(=O)R¹, C(R¹)₂-C(R¹)₂, C(R¹)₂-C(R¹)₂-C(R¹)₂, C(R¹)₂-O und C(R¹)₂-O-C(R¹)₂.

4. Verwendung von Verbindungen gemäß mindestens einem der Ansprüche 1 bis 3 in organischen elektronischen Vorrichtungen.

5. Organische elektronische Vorrichtung, enthaltend mindestens eine Verbindung gemäß mindestens einem der Ansprüche 1 bis 3.

6. Organische elektronische Vorrichtung gemäß Anspruch 5, ausgewählt aus der Gruppe bestehend aus organischen Elektrolumineszenzvorrichtungen (OLEDs, PLEDs), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Laserdioden (O-Laser) oder organischen Photorezeptoren.

7. Organische elektronische Vorrichtung gemäß Anspruch 5, **dadurch gekennzeichnet, dass** sie eine organische Elektrolumineszenzvorrichtung ist, enthaltend Anode, Kathode und mindestens eine emittierende Schicht, **dadurch gekennzeichnet, dass** mindestens eine Verbindung gemäß mindestens einem der Ansprüche 1 bis 3 in einer emittierenden Schicht als Mischung mit mindestens einem fluoreszierendem oder phosphoreszierendem Dotanden eingesetzt wird und/oder dass mindestens eine Verbindung gemäß mindestens einem der Ansprüche 1 bis 3 in einer emittierenden Schicht als Mischung mit mindestens einem Hostmaterial eingesetzt wird.

8. Organische elektronische Vorrichtung gemäß Anspruch 6 oder 7, **dadurch gekennzeichnet, dass** die Verbindung gemäß mindestens einem der Ansprüche 1 bis 3 als Lochtransportmaterial in einer Lochtransportschicht und/oder einer Lochinjektionsschicht eingesetzt wird und/oder dass die Verbindung gemäß mindestens einem der Ansprüche 1 bis 3 als Elektronentransportmaterial in einer Elektronentransportschicht eingesetzt wird.

## Claims

1. Compounds of the formula (2a), (3a) or (4a) 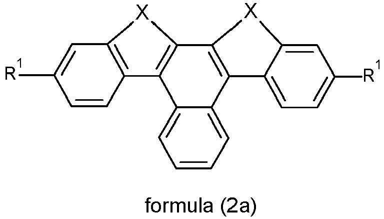 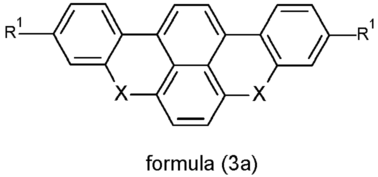 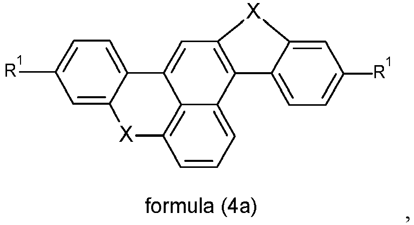 where the following applies to the symbols and indices used:
R¹ stands on each occurrence, identically or differently, for H, F, C(=O)Ar, P(=O)(Ar)₂, CR²=CR²Ar, a straight-chain alkyl group having 1 to 5 C atoms or branched alkyl group having 3 to 5 C atoms, where one or more non-adjacent CH₂ groups may be replaced by -R²C=CR²-, -C≡C- or -O- and where one or more H atoms may be replaced by F, or an aryl group having 6 to 16 C atoms or heteroaryl group having 2 to 16 C atoms or a spirobifluorene group, which may in each case be substituted by one or more radicals R², or a combination of two or three of these systems;
Ar is on each occurrence an aromatic or heteroaromatic ring system having 5-30 aromatic ring atoms, which may be substituted by one or more non-aromatic radicals R¹;
R² is on each occurrence, identically or differently, H or an aliphatic, aromatic or heteroaromatic hydrocarbon radical having 1 to 20 C atoms;
X is, differently on each occurrence, a divalent bridge selected from C(R¹)₂, C=O, C=NR¹, O, S, S=O, SO₂, N(R¹), P(R¹), P(=O)R¹, C(R¹)₂-C(R¹)₂, C(R¹)₂-C(R¹)₂-C(R¹)₂, C(R¹)₂-O and C(R¹)₂-O-C(R¹)₂;
where the naphthalene group may be substituted by one or more radicals R¹,
**characterised in that** at least one substituent R¹ that contains at least one aryl or heteroaryl group is present.

2. Compounds according to Claim 1, **characterised in that** R¹ is selected on each occurrence, identically or differently, from H, aryl groups having 6 to 16 C atoms and heteroaryl groups having 2 to 16 C atoms, where the aryl groups and the heteroaryl groups may in each case be substituted by one or more radicals R².

3. Compounds according to Claim 1 or 2, **characterised in that** the radicals R¹ shown in formulae (2a), (3a) and (4a) are both equal to H and the naphthalene group is not substituted by radicals R¹, and at least one X in the formula selected from formulae (2a), (3a) and (4a) is selected from C(R¹)₂, C=NR¹, N(R¹), P(R¹), P(=O)R¹, C(R¹)₂-C(R¹)₂, C(R¹)₂-C(R¹)₂-C(R¹)₂, C(R¹)₂-O and C(R¹)₂-O-C(R¹)₂.

4. Use of compounds according to at least one of Claims 1 to 3 in organic electronic devices.

5. Organic electronic device containing at least one compound according to at least one of Claims 1 to 3.

6. Organic electronic device according to Claim 5, selected from the group consisting of organic electroluminescent devices (OLEDs, PLEDs), organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), light-emitting electrochemical cells (LECs), organic laser diodes (O-lasers) or organic photoreceptors.

7. Organic electronic device according to Claim 5, **characterised in that** it is an organic electroluminescent device comprising anode, cathode and at least one emitting layer, **characterised in that** at least one compound according to at least one of Claims 1 to 3 is employed in an emitting layer as a mixture with at least one fluorescent or phosphorescent dopant and/or **in that** at least one compound according to at least one of Claims 1 to 3 is employed in an emitting layer as a mixture with at least one host material.

8. Organic electronic device according to Claim 6 or 7, **characterised in that** the compound according to at least one of Claims 1 to 3 is employed as hole-transport material in a hole-transport layer and/or a hole-injection layer and/or **in that** the compound according to at least one of Claims 1 to 3 is employed as electron-transport material in an electron-transport layer.

## Revendications

1. Composés de formule (2a), (3a) ou (4a) 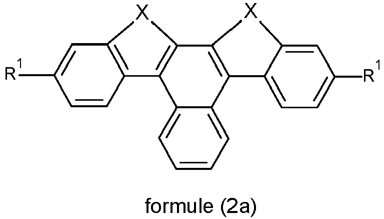 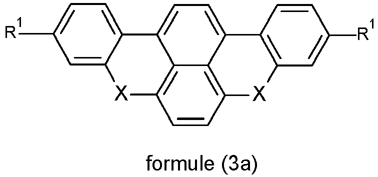 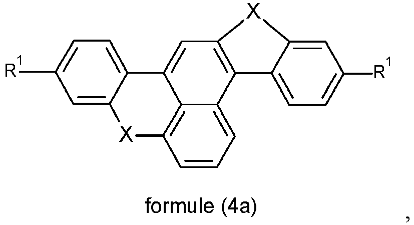 dans laquelle ce qui suit s'applique aux symboles et indices utilisés :
R¹ représente à chaque occurrence, de manière identique ou différente, H, F, C(=O)Ar, P(=O)(Ar)₂, CR²=CR²Ar, un groupement alkyle à chaîne linéaire ayant de 1 à 5 atomes de C ou un groupement alkyle ramifié ayant de 3 à 5 atomes de C, où un ou plusieurs groupements CH₂ non adjacents peuvent être remplacés par -R²C=CR²-, -C≡C- ou -O- et où un ou plusieurs atomes de H peuvent être remplacés par F, ou un groupement aryle ayant de 6 à 16 atomes de C ou un groupement hétéroaryle ayant de 2 à 16 atomes de C ou un groupement spirobi-fluorène, qui peut dans chaque cas être substitué par un ou plusieurs radicaux R², ou une combinaison de deux ou trois parmi ces systèmes ;
Ar est à chaque occurrence un noyau aromatique ou hétéroaromatique ayant 5-30 atomes de cycle aromatique, qui peut être substitué par un ou plusieurs radicaux non aromatiques R¹ ;
R² est à chaque occurrence, de manière identique ou différente, H ou un radical hydrocarboné aliphatique, aromatique ou hétéroaromatique ayant de 1 à 20 atomes de C ;
X est, de manière différente à chaque occurrence, un pont divalent choisi parmi C(R¹)₂, C=O, C=NR¹, O, S, S=O, SO₂, N(R¹), P(R¹), P(=O)R¹, C(R¹)₂-C(R¹)₂, C(R¹)₂-C(R¹)₂-C(R¹)₂, C(R¹)₂-O et C(R¹)₂-O-C(R¹)₂ ;
où le groupement naphtalène peut être substitué par un ou plusieurs radicaux R¹,
**caractérisés en ce qu'**au moins un substituant R¹ qui contient au moins un groupement aryle ou hétéroaryle est présent.

2. Composés selon la revendication 1, **caractérisés en ce que** R¹ est choisi à chaque occurrence, de manière identique ou différente, parmi H, des groupements aryle ayant de 6 à 16 atomes de C et des groupements hétéroaryle ayant de 2 à 16 atomes de C, où les groupements aryle et les groupements hétéroaryle peuvent dans chaque cas être substitués par un ou plusieurs radicaux R².

3. Composés selon la revendication 1 ou 2, **caractérisés en ce que** les radicaux R¹ illustrés dans les formules (2a), (3a) et (4a) sont tous deux égaux à H et le groupement naphtalène n'est pas substitué par des radicaux R¹, et au moins un X dans la formule choisie parmi les formules (2a), (3a) et (4a) est choisi parmi C(R¹)₂, C=NR¹, N(R¹), P(R¹), P(=O)R¹, C(R¹)₂-C(R¹)₂, C(R¹)₂-C(R¹)₂-C(R¹)₂, C(R¹)₂-O et C(R¹)₂-O-C(R¹)₂.

4. Utilisation de composés selon au moins l'une parmi les revendications 1 à 3, dans des dispositifs électroniques organiques.

5. Dispositif électronique organique contenant au moins un composé selon au moins l'une parmi les revendications 1 à 3.

6. Dispositif électronique organique selon la revendication 5, choisi dans le groupe constitué par les dispositifs électroluminescents organiques (OLED, PLED), les transistors organiques à effet de champ (O-FET), les transistors organiques à couche mince (O-TFT), les transistors organiques émetteurs de lumière (O-LET), les circuits intégrés organiques (O-IC), les cellules solaires organiques (O-SC), les dispositifs organiques à extinction de champ (O-FQD), les cellules électrochimiques émettrices de lumière (LEC), les diodes laser organiques (O-lasers) ou les photorécepteurs organiques.

7. Dispositif électronique organique selon la revendication 5, **caractérisé en ce qu'**il s'agit d'un dispositif électroluminescent organique, comprenant une anode, une cathode et au moins une couche émettrice, **caractérisé en ce qu'**au moins un composé selon au moins l'une parmi les revendications 1 à 3 est employé dans une couche émettrice comme mélange avec au moins un dopant fluorescent ou phosphorescent et/ou **en ce qu'**au moins un composé selon au moins l'une parmi les revendications 1 à 3 est employé dans une couche émettrice comme mélange avec au moins un matériau hôte.

8. Dispositif électronique organique selon la revendication 6 ou 7, **caractérisé en ce que** le composé selon au moins l'une parmi les revendications 1 à 3 est employé comme matériau de transport de trous dans une couche de transport de trous et/ou une couche d'injection de trous et/ou **en ce que** le composé selon au moins l'une parmi les revendications 1 à 3 est employé comme matériau de transport d'électrons dans une couche de transport d'électrons.
